# EUROPEAN PATENT SPECIFICATION

(11) **EP 4 672 142 B1**
(45) Date of publication and mention of the grant of the patent: **16.09.2026**
(21) Application number: 25184784.4
(22) Date of filing: 24.06.2025
(51) Int. Cl.: G06T 7/00, A61B 5/02, A61B 6/50

(54) **METHOD, SYSTEM AND DEVICE FOR PERFORMING MEASUREMENT AND CLASSIFICATION FOR CT-FFR, AND STORAGE MEDIUM**
VERFAHREN, SYSTEM UND VORRICHTUNG ZUR DURCHFÜHRUNG VON MESSUNGEN UND KLASSIFIZIERUNGEN FÜR CT-FFR UND SPEICHERMEDIUM
PROCÉDÉ, SYSTÈME ET DISPOSITIF POUR EFFECTUER UNE MESURE ET UNE CLASSIFICATION POUR TOMODENSITOMÉTRIE (TDM-FFR) ET SUPPORT D'INFORMATIONS

(30) Priority: 27.06.2024 CN 202410852486
(43) Date of publication of application: 31.12.2025
(73) Proprietor: Siemens Healthineers AG, 91301 Forchheim (DE)
(72) Inventor: YAN, Jing, Shanghai, 201111 (CN)
(74) Representative: HKW Intellectual Property PartG mbB

(56) References cited:
- TSUGU T ET AL: "Effects of stenotic lesion morphology on trans-lesional computed tomography derived fractional flow reserve (FFRCT) changes", EUROPEAN HEART JOURNAL, vol. 44, no. Supplement_2, 9 November 2023 (2023-11-09), GB, XP093334735, ISSN: 0195-668X, DOI: 10.1093/eurheartj/ehad655.157
- NØRGAARD BJARNE L. ET AL: "Coronary CT Angiography-derived Fractional Flow Reserve Testing in Patients with Stable Coronary Artery Disease: Recommendations on Interpretation and Reporting", RADIOLOGY: CARDIOTHORACIC IMAGING, vol. 1, no. 5, 1 December 2019 (2019-12-01), pages e190050, XP093334739, ISSN: 2638-6135, DOI: 10.1148/ryct.2019190050
- TAEKKER MADSEN KRISTIAN ET AL: "Coronary CT angiography-derived fractional flow reserve in-stable angina: association with recurrent chest pain", vol. 23, no. 11, 18 October 2021 (2021-10-18), Oxford, pages 1511 - 1519, XP093334734, ISSN: 2047-2404, Retrieved from the Internet <URL:https://academic.oup.com/ehjcimaging/article-pdf/23/11/1511/46583599/jeab198.pdf> DOI: 10.1093/ehjci/jeab198
- NOBUO TOMIZAWA: "What Is the Optimal Measurement Method for CT-Derived Fractional Flow Reserve?", ECHOCARDIOGRAPHY, FUTURA PUBLISHING CO., ARMONK, NY, US, vol. 42, no. 4, 8 April 2025 (2025-04-08), pages n/a, XP072870053, ISSN: 0742-2822, DOI: 10.1111/ECHO.70161

## Description

### TECHNICAL FIELD

The present invention relates to a method, system and device for performing measurement and classification for CT-FFR, and a storage medium.

### BACKGROUND ART

CT-FFR (Computed Tomography Fractional Flow Reserve), as a non-invasive technique for assessing functional stenosis in coronary artery disease, has good correlation with invasive FFR (Fractional Flow Reserve) in terms of diagnostic performance, but nonetheless has certain drawbacks. Firstly, manually selected measurement positions may introduce subjectivity, affecting the uniformity of results. Secondly, CT-FFR values within the range of 2 cm - 3 cm distal to the stenosis might vary due to differences in centerline points and lumen contour, resulting in uncertainty of measurement results. In addition, when the CT-FFR value lies in a "gray zone" between 0.75 and 0.8, it is of little diagnostic value, and might lead to misjudgment.

The statement here merely provides background art related to the present invention, and does not necessarily form prior art.

Tsugu, Toshimitsu & Tanaka, Kaoru & Nagatomo, Yuji & Belsack, Dries & Argacha, J & Cosyns, Bernard & Maeseneer, M & De Mey, Johan. (2023). Effects of stenotic lesion morphology on trans-lesional computed tomography derived fractional flow reserve (FFRCT) changes. European Heart Journal. 44. 10.1093/eurheartj/ehad655.157, investigates the effect of lesion morphology on ΔFFRCT and the ability of ΔFFRCT to detect lesions with the coronary flow disturbance. A total of 1502 outpatients with suspected stable coronary artery disease were evaluated. Among them, 76 patients who underwent both FFRCT and invasive coronary angiography with > 50% stenosis were evaluated and divided into four groups according to the degree of stenosis and lesion length and lesion length. Vessel and lesion morphology (length, luminal volume, and plaque characteristics) and left ventricular mass were assessed. Indication for revascularization (percutaneous coronary intervention and coronary artery bypass graft) was assessed by invasive coronary angiography and additional invasive FFR measurement was performed when necessary. The study concludes that ΔFFRCT was affected by lesion length, followed by lesion calcium components. ΔFFRCT predicted lesions requiring revascularization.

Norgaard BL, Fairbairn TA, Safian RD, Rabbat MG, Ko B, Jensen JM, Nieman K, Chinnaiyan KM, Sand NP, Matsuo H, Leipsic J, Raff G. Coronary CT Angiography-derived Fractional Flow Reserve Testing in Patients with Stable Coronary Artery Disease: Recommendations on Interpretation and Reporting. Radiol Cardiothorac Imaging. 2019 Nov 21;1(5):e190050. doi: 10.1148/ryct.2019190050. PMID: 33778528; PMCID: PMC7977999, discloses that noninvasive fractional flow reserve derived from coronary CT angiography (FFRCT) is increasingly used in patients with coronary artery disease as a gatekeeper to the catheterization laboratory. While there is emerging evidence of the clinical benefit of FFRCT in patients with moderate coronary disease as determined with coronary CT angiography, there has been less focus on interpretation, reporting, and integration of FFRCT results into routine clinical practice. Because FFRCT analysis provides a plethora of information regarding pressure and flow across the entire coronary tree, standardized criteria on interpretation and reporting of the FFRCT analysis result are of crucial importance both in context of the clinical adoption and in future research. The report represents expert opinion and recommendation on a standardized FFRCT interpretation and reporting approach.

Tækker Madsen K, Veien KT, Larsen P, Husain M, Deibjerg L, Junker A, Kusk MW, Thomsen KK, Rohold A, Jensen LO, Sand NPR. Coronary CT angiography-derived fractional flow reserve in-stable angina: association with recurrent chest pain. Eur Heart J Cardiovasc Imaging. 2022 Oct 20;23(11):1511-1519. doi: 10.1093/ehjci/jeab198. PMID: 34661645; PMCID: PMC9584620, aims to evaluate the association between coronary computed tomography angiography (CCTA)-derived fractional flow reserve (FFRCT) and recurrent chest pain (CP) at 1-year follow-up in patients with stable angina pectoris (SAP), and concludes that an abnormal FFRCT test result is associated with an increased risk of recurrent CP in patients with new-onset SAP.

Tomizawa N. What Is the Optimal Measurement Method for CT-Derived Fractional Flow Reserve? Echocardiography. 2025 Apr;42(4):e70161. doi: 10.1111/echo.70161. PMID: 40198607, discloses that CT-FFR is a promising method to noninvasively diagnose functionally significant coronary stenosis. However, factors such as measurement methods, lesion type, and lesion length need to be considered to make a clinically relevant diagnosis.

### SUMMARY OF THE INVENTION

An object of the present invention is to provide a method, system and device for performing measurement and classification for CT-FFR, and a storage medium, which improve measurement accuracy, enhance the accuracy and stability of assessment, reduce interference, and provide a more reliable solution for clinical physicians and patients.

To achieve the above object, the present invention provides a method for performing measurement and classification for Computed Tomography Fractional Flow Reserve, comprising:
dividing lesions into different lesion types, according to branch involvement and calcification and length of lesions displayed in a Coronary Computed Tomography Angiography (CCTA) image;
determining focus lengths of lesion positions according to the different lesion types;
determining measurement ranges for Computed Tomography Fractional Flow Reserve according to the focus lengths of the different lesion types;
obtaining all Computed Tomography Fractional Flow Reserve (CT-FFR) values within the measurement ranges, subjecting all the CT-FFR values to statistical calculation, and performing lesion specific classification of calculation results according to cut-off values from hemodynamical classification, to obtain a lesion specific classification result.

A lesion type classification function is provided in this solution, being able to identify lesions of different types, select a suitable focus length measurement strategy according to the different lesion types, and thereby determine measurement ranges for CT-FFR values of specific lesions, so as to increase the accuracy of measurement. By performing lesion specific classification of lesions in a hemodynamical sense, the accuracy and stability of CT-FFR assessment is enhanced, interference is reduced, and a more reliable solution is provided for clinical physicians and patients.

Optionally, the method of automatically dividing lesion types comprises:
if the length of a lesion is less than or equal to 10 mm, with mild or no calcification, and no principal branch involvement, then it is determined as being a single focal lesion;
if the length of a lesion is greater than or equal to 20 mm, with principal branch involvement, and excessive proximal twisting, then it is determined as being a diffuse lesion;
if the length of an entire lesion is more than 10 mm but less than 20 mm, with moderate or severe calcification, and an irregular contour, then it is determined as being a serial lesion; if lesions are spaced apart by less than 10 mm, then they are determined as being a continuous serial lesion, and if lesions are spaced apart by 10 mm or more, then they are determined as being a discontinuous serial lesion.

In this solution, by identifying lesions of different types such as single focal lesions, diffuse lesions and serial lesions, a suitable focus length measurement strategy can be chosen according to different lesion types, and measurement ranges for CT-FFR values of specific lesions can be determined, so as to increase the accuracy of measurement.

Optionally, the method of determining focus lengths of lesion positions comprises:
for a single focal lesion, separately recording a lesion proximal centerline point LPₚᵣₒ and a lesion distal centerline point LP_{dis};
for a diffuse lesion, separately recording a lesion proximal centerline point LPₚᵣₒ and a lesion distal centerline point LP_{dis};
for a serial lesion, first determining a proximal centerline point LPₚᵣₒ and a lesion distal centerline point LP_{dis} of the entire lesion, then determining continuity of the entire lesion, to determine whether the serial lesion is a continuous serial lesion or a discontinuous serial lesion;
for a continuous serial lesion, separately recording a lesion proximal centerline point LPₚᵣₒ and a lesion distal centerline point LP_{dis};
for a discontinuous serial lesion, first recording a lesion proximal centerline point LPₚᵣₒ and a lesion distal centerline point LP_{dis} of the entire lesion, then separately recording a lesion proximal centerline point LPₚᵣₒ' and a lesion distal centerline point LP_{dis}' of each lesion.

In this solution, based on the different lesion types, a matching method is chosen to determine the lesion proximal centerline point and lesion distal centerline point, so as to determine measurement ranges for CT-FFR values of specific lesions according to different focus lengths, in order to increase measurement accuracy.

Optionally, for single focal lesions, diffuse lesions and continuous serial lesions, the method of determining the measurement range for Computed Tomography Fractional Flow Reserve comprises:
calculating a centerline point MP_{beg} for the start of measurement and a centerline point MP_{end} for the end of measurement: ${MP}_{beg} = {LP}_{dis} + 2 cm$ wherein the centerline point MP_{beg} for the start of measurement is 2 cm longitudinally along the coronary artery from the lesion distal centerline point LP_{dis} in the lesion distal direction; ${MP}_{end} = {LP}_{dis} + 3 cm$ wherein the centerline point MP_{end} for the end of measurement is 3 cm longitudinally along the coronary artery from the lesion distal centerline point LP_{dis} in the lesion distal direction.

In this solution, the centerline point MP_{beg} for the start of measurement and the centerline point MP_{end} for the end of measurement are determined according to the different lesion types and lesion lengths, reducing interference, increasing accuracy, and helping physicians to learn the exact length and position of lesions.

Optionally, for discontinuous serial lesions, the method of determining the measurement range for Computed Tomography Fractional Flow Reserve comprises:
calculating a centerline point MP_{beg} for the start of measurement and a centerline point MP_{end} for the end of measurement: ${MP}_{beg} = {CLP}_{normal from {LP}_{dis}}$ wherein the centerline point MP_{end} for the start of measurement is the position of the centerline point where normal blood vessel diameter is restored from the previous lesion; ${MP}_{end} = {MP}_{beg} + \left(0.5 to 1\right) mm$ wherein the centerline point MP_{end} for the end of measurement is 0.5 mm - 1 mm longitudinally along the coronary artery from the centerline point MP_{beg} for the start of measurement in the lesion distal direction.

In this solution, the centerline point MP_{beg} for the start of measurement and the centerline point MP_{end} for the end of measurement are determined according to the different lesion types and lesion lengths, reducing interference, increasing accuracy, and helping physicians to learn the exact length and position of lesions.

Optionally, if a distal end of a lesion is close to the farthest end of the coronary artery, then a centerline point at a position where lumen diameter is 1.5 mm is taken to be the lesion distal centerline point when determining the focus length of the lesion position, and a centerline point at a position where lumen diameter is 1.5 mm is taken to be the centerline point MP_{end} for the end of measurement when determining the measurement range for Computed Tomography Fractional Flow Reserve: MP_{end} = 1.5 mm lumen diameter centerline point.

In this solution, a typical or average diameter of the distal end of a lesion is generally 1.5 mm, so choosing this point as a reference can help a physician to assess the length and severity of the lesion more accurately.

Optionally, the method of performing lesion specific classification comprises:
when the maximum value of all the CT-FFR values within the measurement range is less than or equal to a cut-off value 0.7, the mean value of all the CT-FFR values within the measurement range is used as the CT-FFR value of the lesion, and the lesion is labeled as positive;
when the minimum value of all the CT-FFR values within the measurement range is greater than or equal to a cut-off value 0.85, the mean value of all the CT-FFR values within the measurement range is used as the CT-FFR value of the lesion, and the lesion is labeled as negative;
when there are CT-FFR values within the measurement range that are distributed between 0.7 and 0.85, a histogram is established on the basis of all the CT-FFR values, a category with the highest frequency in the histogram is determined, and the mean value of CT-FFR values in this category is calculated and compared with the cut-off values; if the mean value is less than or equal to 0.7, the lesion is determined as being a positive lesion, and labeled as positive; if the mean value is greater than or equal to 0.85, the lesion is determined as being a negative lesion, and labeled as negative; if the mean value is greater than 0.7 but less than 0.85, the lesion is determined as being in a gray zone.

In this solution, lesion specific classification of lesions in a hemodynamical sense is performed by a histogram statistical method, displaying the distribution characteristics of CT-FFR values in a visually direct and effective way, thus enhancing the accuracy and stability of CT-FFR assessment, reducing interference, and providing a more reliable solution for clinical physicians and patients.

Another technical solution of the present invention provides a system for performing measurement and classification for Computed Tomography Fractional Flow Reserve, used to realize the method for performing measurement and classification for Computed Tomography Fractional Flow Reserve, the system comprising:
a lesion type classification module, for dividing lesions into different types, according to branch involvement and calcification and length of lesions displayed in a Coronary Computed Tomography Angiography (CCTA) image;
a focus length determining module, for determining focus lengths of lesion positions according to the different lesion types;
a measurement range determining module, for determining measurement ranges for Computed Tomography Fractional Flow Reserve according to the focus lengths of the different lesion types;
a CT-FFR value calculating module, for calculating all Computed Tomography Fractional Flow Reserve (CT-FFR) values within the measurement ranges;
a statistical analysis module, for subjecting the Computed Tomography Fractional Flow Reserve values to statistical analysis according to the measurement ranges, to obtain a lesion specific classification result.

In this solution, a lesion type classification function is provided, being able to identify lesions of different types, select a suitable focus length measurement strategy according to the different lesion types, and thereby determine measurement ranges for CT-FFR values of specific lesions, so as to increase the accuracy of measurement. By performing lesion specific classification of lesions in a hemodynamical sense, the accuracy and stability of CT-FFR assessment is enhanced, interference is reduced, and a more reliable solution is provided for clinical physicians and patients.

Another technical solution of the present invention provides a device for performing measurement and classification for Computed Tomography Fractional Flow Reserve, comprising: a memory, a processor, and a program stored on the memory and able to run on the processor, the program being configured to realize the method for performing measurement and classification for Computed Tomography Fractional Flow Reserve.

Another technical solution of the present invention provides a storage medium, wherein a program is stored on the storage medium, and the program, when executed by a processor, realizes the method for performing measurement and classification for Computed Tomography Fractional Flow Reserve.

In summary, the present invention provides a lesion type classification function, being able to identify lesions of different types such as single focal lesions, diffuse lesions and serial lesions, select a suitable focus length measurement strategy according to the different lesion types, and thereby determine measurement ranges for CT-FFR values of specific lesions, so as to increase the accuracy of measurement. By performing lesion specific classification of lesions in a hemodynamical sense, the accuracy and stability of CT-FFR assessment is enhanced, interference is reduced, and a more reliable solution is provided for clinical physicians and patients.

### BRIEF DESCRIPTION OF THE DRAWINGS

Fig. 1 is a flow chart of a method provided in an embodiment of the present invention for performing measurement and classification for CT-FFR.
Fig. 2 is a schematic picture showing the determination of focus lengths and measurement ranges of a single focal lesion, a diffuse lesion and a continuous serial lesion.
Fig. 3 is a schematic picture showing the determination of focus length and measurement range of a discontinuous serial lesion.
Fig. 4 is a block diagram of a system provided in an embodiment of the present invention for performing measurement and classification for CT-FFR.

Reference numerals used in the drawings:
S1 - S4: steps of a method provided in an embodiment for performing measurement and classification for CT-FFR;
LPₚᵣₒ: lesion proximal centerline point of entire lesion;
LP_{dis}: lesion distal centerline point of entire lesion;
LPₚᵣₒ': lesion proximal centerline point of each lesion in a discontinuous serial lesion;
LP_{dis}': lesion distal centerline point of each lesion in a discontinuous serial lesion;
MP_{beg}: centerline point for start of measurement;
MP_{end}: centerline point for end of measurement;
101: lesion type classification module;
102: focus length determining module;
103: measurement range determining module;
104: CT-FFR value calculating module;
105: statistical analysis module.

### DETAILED DESCRIPTION OF THE INVENTION

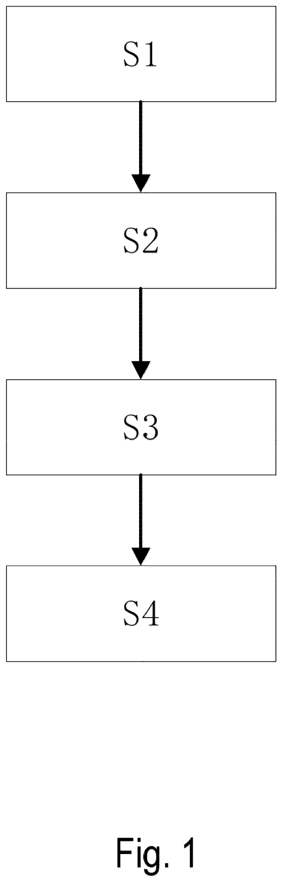

Preferred embodiments of the present invention are explained in detail below with reference to Figs. 1 - 4.

Fractional Flow Reserve (FFR) is an important reference standard for clinical evaluation of functional stenosis in coronary artery disease (CAD). FFR is calculated by measuring the ratio of distal coronary artery pressure (Pd) to aortic pressure (Pa) in a state of maximum coronary artery congestion, and has a value in the range of 0.0 to 1.0. The measurement of FFR is typically performed under the guidance of Percutaneous Coronary Intervention (PCI), and it is necessary to use a pressure guide wire and to ensure that a pressure sensor is positioned at least 2 cm - 3 cm distal to the stenosis under assessment. It is generally considered that lesions with an FFR value ≤ 0.8 will cause lesion-specific ischemia, while lesions with an FFR value > 0.8 have little association with myocardial ischemia. When the FFR value is between 0.75 and 0.8, it is considered to be in the "gray zone", and is of little diagnostic value.

When measuring FFR, it is necessary to closely monitor blood pressure and congestion in the patient; measurement is carried out when the lowest point level of the Pd/Pa trace has remained stable for 20 seconds after pressure system calibration, balancing of the distal coronary artery pressure Pd and aortic pressure Pa, and injection of adenosine. In the case of multiple continuous stenosis or diffuse atherosclerosis disease, it is necessary to slowly pull back the pressure sensor in a stable congestion state to assess the distribution of abnormal epicardial resistance, and examine the entire length of the artery within a time period of approximately 15 - 20 seconds.

Computed Tomography Fractional Flow Reserve (CT-FFR) is a non-invasive assessment technique, which is based on Coronary Computed Tomography Angiography (CCTA) data combined with an advanced computational analysis method such as computational fluid dynamics (CFD) or machine learning (ML). CT-FFR aims to simulate the physiological state of the coronary artery, providing simulated values similar to those of invasive FFR in order to assess the hemodynamical significance of coronary artery lesions. CT-FFR has good correlation with invasive FFR in terms of diagnostic performance, and compared with CCTA alone, shows potential with regard to optimizing patient management, improving risk stratification and prognosis.

The computation process of CT-FFR includes: using CCTA data to reconstruct an anatomical model of the coronary arteries, extracting coronary artery centerlines and lumen contour lines, then inputting the extracted coronary artery centerline tree, performing corresponding lumen segmentation at each centerline point, and using a machine learning algorithm to predict CT-FFR values of each centerline point along the centerline tree, to finally generate a color coded grid to display the CT-FFR values corresponding to each centerline point.

The CT-FFR measurement position is generally recommended to be 2 cm - 3 cm distal to the stenosis, so as to have better correlation with invasive FFR. However, since the CT-FFR measurement position is chosen manually, and CT-FFR values within the range of 2 cm - 3 cm distal to the stenosis might vary due to differences in centerline points and lumen contour, this might cause uncertainty or non-uniformity of measurement; in particular, when the CT-FFR values are in the gray zone, it may even cause misjudgments.

When a software algorithm is applied to perform automated detection and measurement, a fixed centerline point (e.g. a position 2 cm distal to the lesion) will generally be chosen as the measurement point for CT-FFR values. Although such a measurement method is simple, convenient and quick in operation, and capable of obtaining a reference value rapidly, measurement at a fixed center point might neglect minute changes in actual blood flow in the lesion area, and a fixed center point alone might be insufficient to accurately reflect CT-FFR values for the entire lesion area, and unable to fully take into account the complexity and diversity of lesions, so the precision and accuracy of measurement is reduced. Therefore, when choosing a measurement position for CT-FFR, lesions of different types should be taken into account together, and the measurement position should be chosen as precisely as possible, to ensure clinical relevance and accuracy of the measurement result.

In clinical practice, there are clear guiding principles for the selection of CT-FFR measurement position, based on lesion type and distribution: for a single focal lesion, the measurement point is typically chosen manually 2 cm - 3 cm distal to the stenosis; for a diffuse lesion of a length greater than or equal to 3 cm, or a continuous serial lesion with a spacing of less than or equal to 1 cm, measurement is performed 2 cm distal to the lesion; for an intermittent serial lesion with a spacing greater than or equal to 1 cm, it is recommended that measurement be performed at the distal end of each lesion. For the majority of stenosis lesions, the measurement points of distal epigenetically normal coronary arteries should extend from the distal end of the stenosis to 2 cm from the entire lesion.

On this basis, the present invention provides a method for performing automatic measurement and classification for CT-FFR; as shown in Fig. 1, the method comprises the following steps:
Step S1, dividing lesions into different lesion types, according to branch involvement and calcification and length of lesions displayed in a Coronary Computed Tomography Angiography (CCTA) image.
Step S2, determining focus lengths of lesion positions according to the different lesion types.
Step S3, determining measurement ranges for Computed Tomography Fractional Flow Reserve according to the focus lengths of the different lesion types.
Step S4, obtaining all Computed Tomography Fractional Flow Reserve (CT-FFR) values within the measurement ranges, subjecting all the CT-FFR values to statistical calculation, and performing lesion specific classification of calculation results according to cut-off values from hemodynamical classification, to obtain a lesion specific classification result.

The present invention provides a lesion type classification function, being able to identify lesions of different types, select a suitable focus length measurement strategy according to the different lesion types, and thereby customize measurement ranges for CT-FFR values of specific lesions, so as to increase the accuracy of measurement. By performing lesion specific classification of lesions in a hemodynamical sense, the accuracy and stability of CT-FFR assessment is enhanced, interference is reduced, and a more reliable solution is provided for clinical physicians and patients.

In step S1, the lesion types include: single focal lesions, diffuse lesions and serial lesions, wherein serial lesions are further divided into continuous serial lesions and discontinuous serial lesions.

In step S1, the method of dividing lesion types comprises:
measuring lesion lengths in the CCTA image, and analysing branch involvement and degree of calcification of lesions in the CCTA image.

The Agatston score is commonly used clinically to assess the severity of calcification of the coronary arteries; generally, scores of 0, 1 - 100, 101 - 400, and more than 400 for calcification of the coronary arteries respectively correspond to four categories: no calcification, slight calcification, moderate calcification, and severe calcification.

Single focal lesions: if the length of a lesion is less than or equal to 10 mm, with mild or no calcification, and no principal branch involvement, then it is determined as being a single focal lesion. Single focal lesions also have other characteristics, such as incomplete occlusion (meaning that the blood vessel has not been blocked completely, and there is still some blood flowing through it), the position is not at an opening (the lesion is not located at an opening of the blood vessel, i.e. a starting region of a blood vessel branch), there is no thrombus (there is no thrombosis in the blood vessel), and the contour is smooth. According to the classification of coronary artery lesions by the American College of Cardiology (ACC) and the American Heart Association (AHA), single focal lesions are generally classified as typical type A lesions (simple lesions).

Diffuse lesions: if the length of a lesion is greater than or equal to 20 mm, with principal branch involvement, and excessive proximal twisting (meaning that abnormal bending or twisting has occurred in a proximal part of the blood vessel, i.e. a part nearer to the heart), then it is determined as being a diffuse lesion. According to the classification of coronary artery lesions by the American College of Cardiology (ACC) and the American Heart Association (AHA), diffuse lesions are generally classified as typical type C lesions (complex lesions).

Serial lesions: if the length of an entire lesion is more than 10 mm but less than 20 mm, with moderate or severe calcification, and an irregular contour, then it is determined as being a serial lesion; further, if lesions are spaced apart by less than 10 mm, then they are determined as being a continuous serial lesion, and if lesions are spaced apart by 10 mm or more, then they are determined as being a discontinuous serial lesion. According to the classification of coronary artery lesions by the American College of Cardiology (ACC) and the American Heart Association (AHA), serial lesions are generally classified as typical type B lesions (lesions of medium complexity).

By identifying lesions of different types such as single focal lesions, diffuse lesions and serial lesions, a suitable focus length measurement strategy can be chosen according to different lesion types, and measurement ranges for CT-FFR values of specific lesions can be determined automatically, so as to increase the accuracy of measurement.

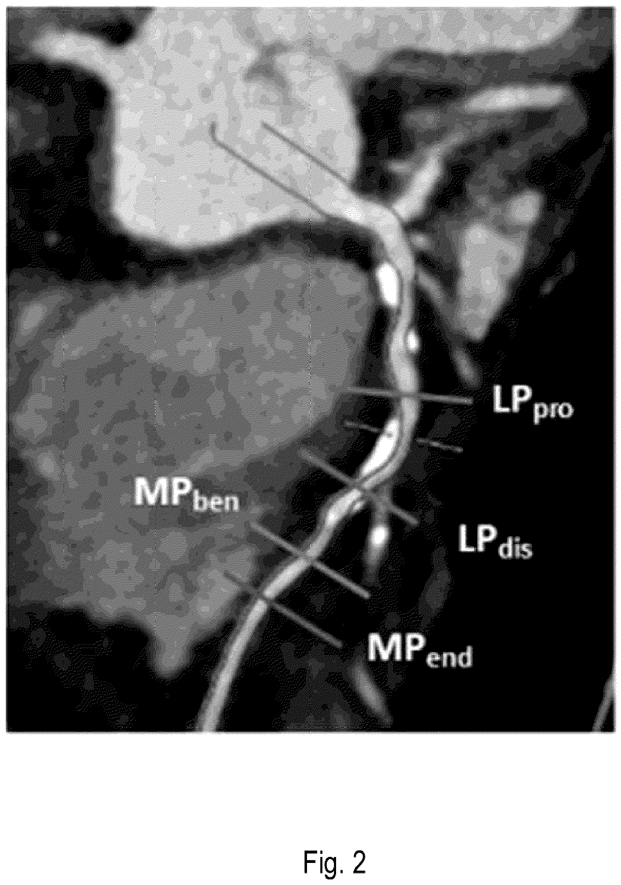

In step S2, the method of determining focus lengths of lesion positions comprises:
As shown in Fig. 2, for a single focal lesion, a lesion proximal centerline point LPₚᵣₒ and a lesion distal centerline point LP_{dis} are separately recorded; if the lesion is close to the farthest end of the coronary artery, then a centerline point at a position where the lumen diameter is 1.5 mm is taken to be the lesion distal centerline point LP_{dis}. A typical or average diameter of the distal end of a lesion is generally 1.5 mm, so choosing this point as a reference can help a physician to assess the length and severity of the lesion more accurately.

As shown in Fig. 2, for a diffuse lesion, a lesion proximal centerline point LPₚᵣₒ and a lesion distal centerline point LP_{dis} are separately recorded; if the lesion is close to the farthest end of the coronary artery, then a centerline point at a position where the lumen diameter is 1.5 mm can be taken to be the lesion distal centerline point LP_{dis}.

For a serial lesion, LPₚᵣₒ and LP_{dis} of the entire lesion are first determined, then determination of continuity is performed, comprising: if there is a sudden change in the rate of change of radius in a middle part of the lesion, i.e. the diameter is greater than at the proximal end of the lesion, and the length is greater than 1 cm, then it is classified as a discontinuous serial lesion.

As shown in Fig. 2, for a continuous serial lesion, a lesion proximal centerline point LPₚᵣₒ and a lesion distal centerline point LP_{dis} are separately recorded; if the lesion is close to the farthest end of the coronary artery, then a centerline point at a position where the lumen diameter is 1.5 mm can be taken to be the lesion distal centerline point LP_{dis}.

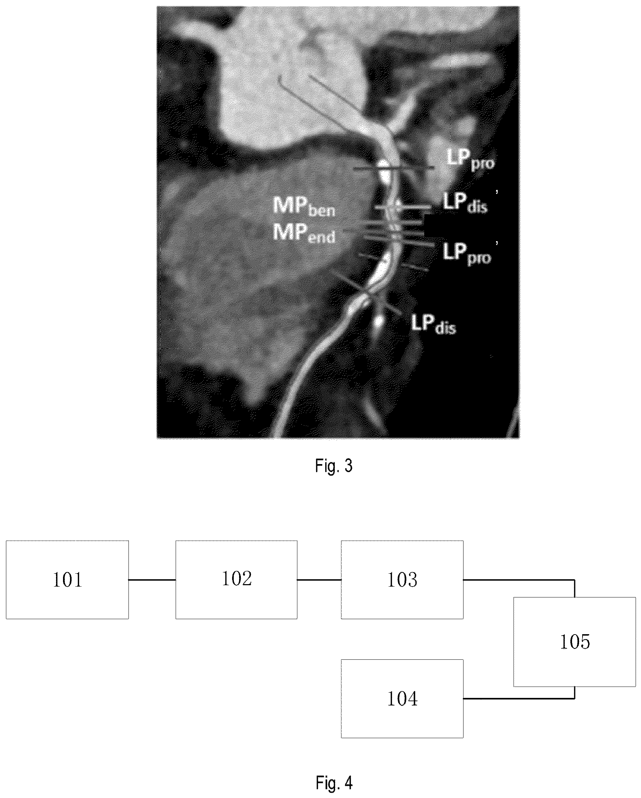

As shown in Fig. 3, for a discontinuous serial lesion, a lesion proximal centerline point LPₚᵣₒ and a lesion distal centerline point LP_{dis} of the entire lesion are recorded first, then a lesion proximal centerline point LPₚᵣₒ' and a lesion distal centerline point LP_{dis}' of each lesion are separately recorded. If the lesion is close to the farthest end of the coronary artery, then a centerline point at a position where the lumen diameter is 1.5 mm can be taken to be the lesion distal centerline point.

Based on the different lesion types, a matching method is chosen to determine the lesion proximal centerline point and lesion distal centerline point, so as to determine measurement ranges for CT-FFR values of specific lesions according to different focus lengths, in order to increase measurement accuracy.

In step S3, the method of determining measurement ranges for Computed Tomography Fractional Flow Reserve comprises:
As shown in Fig. 2, for single focus lesions, diffuse lesions and continuous serial lesions, a centerline point MP_{beg} for the start of measurement and a centerline point MP_{end} for the end of measurement are calculated: ${MP}_{beg} = {LP}_{dis} + 2 cm$ wherein the centerline point MP_{beg} for the start of measurement is 2 cm longitudinally along the coronary artery from the lesion distal centerline point LP_{dis} in the lesion distal direction. ${MP}_{end} = {LP}_{dis} + 3 cm$ wherein the centerline point MP_{end} for the end of measurement is 3 cm longitudinally along the coronary artery from the lesion distal centerline point LP_{dis} in the lesion distal direction.

Determining the centerline point MP_{beg} for the start of measurement and the centerline point MP_{end} for the end of measurement helps the physician to learn the exact length and position of the lesion.

### Alternatively:

If the distal end of the lesion is close to the farthest end of the coronary artery, then a centerline point at a position where the lumen diameter is 1.5 mm is taken to be the centerline point MP_{end} for the end of measurement, ${MP}_{end} = 1.5 mm lumen diameter centerline point$ choosing this point as a reference can help the physician to assess the length and severity of the lesion more accurately.

As shown in Fig. 3, for a discontinuous serial lesion, the centerline point MP_{beg} for the start of measurement and the centerline point MP_{end} for the end of measurement are calculated: ${MP}_{beg} = {CLP}_{normal from {LP}_{dis}}$ wherein the centerline point MP_{beg} for the start of measurement is the position of the centerline point where normal blood vessel diameter is restored from the previous lesion; ${MP}_{end} = {MP}_{beg} + \left(0.5 to 1\right) mm$ wherein the centerline point MP_{end} for the end of measurement is 0.5 mm - 1 mm longitudinally along the coronary artery from the centerline point MP_{beg} for the start of measurement in the lesion distal direction.

Determining the centerline point MP_{beg} for the start of measurement and the centerline point MP_{end} for the end of measurement helps the physician to learn the exact length and position of the lesion.

Alternatively, if the distal end of the lesion is close to the farthest end of the coronary artery, then a centerline point at a position where the lumen diameter is 1.5 mm is taken to be the centerline point MP_{end} for the end of measurement, ${MP}_{end} = 1.5 mm lumen diameter centerline point$ choosing this point as a reference can help the physician to assess the length and severity of the lesion more accurately.

In step S4, the CT-FFR values are statistically analysed according to the measurement ranges, to obtain a lesion specific classification result.

All Computed Tomography Fractional Flow Reserve (CT-FFR) values within the measurement ranges are obtained, statistical matrices are established to subject all the values to calculation, and the calculation results are classified according to cut-off values from hemodynamical classification. The CT-FFR sampling interval and sampling frequency are generally related to CT scan parameter settings, and the specific values might vary for different CT apparatuses and scanning protocols.

The statistical matrix includes: a minimum value, a maximum value, a mean value, and a histogram.

The cut-off values are 0.7 and 0.85; if the CT-FFR value ≤ 0.7, the lesion is determined as being a positive lesion; if the CT-FFR value ≥ 0.85, the lesion is determined as being a negative lesion; if the CT-FFR value is between these two values, the lesion will be determined as being in the gray zone, signifying that further clinical evidence is needed to perform classification.

When the maximum value of all the CT-FFR values within the measurement range is less than or equal to 0.7, the mean value of all the CT-FFR values within the measurement range is used as the CT-FFR value of the lesion, and the lesion is labeled as positive, e.g. labeled red.

When the minimum value of all the CT-FFR values within the measurement range is greater than or equal to 0.85, the mean value of all the CT-FFR values within the measurement range is used as the CT-FFR value of the lesion, and the lesion is labeled as negative, e.g. labeled blue.

When there are CT-FFR values within the measurement range that are distributed between 0.7 and 0.85, a histogram is established on the basis of all the CT-FFR values, a category with the highest frequency in the histogram is determined, and the mean value of CT-FFR values in this category is calculated and compared with the cut-off values; if the mean value is less than or equal to 0.7, the lesion is determined as being a positive lesion, and labeled as positive; if the mean value is greater than or equal to 0.85, the lesion is determined as being a negative lesion, and labeled as negative; if the mean value is greater than 0.7 but less than 0.85, the lesion is determined as being in the gray zone, and labeled as being in the gray zone, e.g. labeled gray.

The use of a histogram to analyse the CT-FFR values provides a visually direct and effective means of visualizing data, which is not only able to clearly display distribution characteristics of the CT-FFR values, including concentration trends, degree of dispersion and data density, but also helps to quickly identify abnormal values and outlying points. Histograms enable physicians to compare CT-FFR value distributions of different patient groups, to assist with diagnostic decisions, and judge whether data is within a normal range. The adjustability of histograms allows physicians to adjust group numbers according to different analysis requirements, to obtain more precise or more general views of data, so as to better assess and interpret CT-FFR data.

The present invention divides lesions into different types according to a Coronary Computed Tomography Angiography (CCTA) image, determines focus lengths of lesion positions according to different lesion types, then determines Computed Tomography Fractional Flow Reserve measurement ranges according to the focus lengths of different lesion types, establishes statistical matrices to subject all Computed Tomography Fractional Flow Reserve (CT-FFR) values within the measurement ranges to calculation, and finally subjects the calculation results to hemodynamical classification.

The present invention introduces a lesion type classification function, being able to automatically identify lesions of different types such as single focal lesions, diffuse lesions and serial lesions, select a suitable focus length measurement strategy according to the different lesion types, and thereby determine measurement ranges for CT-FFR values of specific lesions, so as to increase measurement accuracy. The present invention performs lesion specific classification of lesions in a hemodynamical sense, enhancing the accuracy and stability of CT-FFR assessment, reducing interference, and providing a more reliable solution for clinical physicians and patients.

As shown in Fig. 4, the present invention further provides a system for performing measurement and classification for Computed Tomography Fractional Flow Reserve, comprising:
a lesion type classification module 101, for dividing lesions into different types, according to branch involvement and calcification and length of lesions displayed in a Coronary Computed Tomography Angiography (CCTA) image;
a focus length determining module 102, for determining focus lengths of lesion positions according to the different lesion types;
a measurement range determining module 103, for determining measurement ranges for Computed Tomography Fractional Flow Reserve according to the focus lengths of the different lesion types;
a CT-FFR value calculating module 104, for calculating all Computed Tomography Fractional Flow Reserve (CT-FFR) values within the measurement ranges;
a statistical analysis module 105, for subjecting the Computed Tomography Fractional Flow Reserve values to statistical analysis according to the measurement ranges, to obtain a lesion specific classification result.

The present invention introduces a lesion type classification function, being able to identify lesions of different types such as single focal lesions, diffuse lesions and serial lesions, select a suitable focus length measurement strategy according to the different lesion types, and thereby determine measurement ranges for CT-FFR values of specific lesions, so as to increase measurement accuracy. The present invention performs lesion specific classification of lesions in a hemodynamical sense, enhancing the accuracy and stability of CT-FFR assessment, reducing interference, and providing a more reliable solution for clinical physicians and patients.

The present invention further provides an apparatus for performing measurement and classification for Computed Tomography Fractional Flow Reserve, comprising: a memory, a processor, and a program stored on the memory and able to run on the processor, the program being configured to realize the method for performing measurement and classification for Computed Tomography Fractional Flow Reserve.

The present invention further provides a storage medium, the storage medium having a program stored thereon, wherein the program, when executed by the processor, realizes the method for performing measurement and classification for Computed Tomography Fractional Flow Reserve.

The present invention introduces a lesion type classification function, being able to identify lesions of different types such as single focal lesions, diffuse lesions and serial lesions, select a suitable focus length measurement strategy according to the different lesion types, and thereby determine measurement ranges for CT-FFR values of specific lesions, so as to increase measurement accuracy. The present invention performs lesion specific classification of lesions in a hemodynamical sense, enhancing the accuracy and stability of CT-FFR assessment, reducing interference, and providing a more reliable solution for clinical physicians and patients.

It should be explained that in embodiments of the present invention, orientations or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", "anticlockwise", "axial", "radial" and "circumferential" are based on the orientations or positional relationships shown in the drawings, and are only intended to facilitate the description of embodiments, without indicating or implying that the device or element referred to must have a specific orientation or be constructed and operated in a specific orientation, and therefore must not be construed as limiting the present invention. In addition, the terms "first", "second" and "third" are merely used to describe objects, and must not be construed as indicating or implying relative importance.

In the present invention, unless otherwise clearly specified and defined, terms such as "install", "connected", "connect" and "fix" should be understood in a broad sense, e.g. may mean a fixed connection, a detachable connection or integral forming; a mechanical connection or an electrical connection; a direct connection, an indirect connection via an intermediate medium, an internal connection of two elements, or an interactive relationship between two elements. Those skilled in the art will be able to understand the specific meanings of the abovementioned terms in the present invention according to the specific circumstances.

It will be understood that as used in this description and the appended claims, the term "comprises" indicates the presence of the described feature, entity, step, operation, element and/or assembly, but does not rule out the presence or addition of one or more other feature, entity, step, operation, element, assembly and/or set thereof.

It should also be understood that terms used in the description of the present application herein are for the sole purpose of describing specific embodiments, and are not intended to limit the present application. As used in the description of the present application and the appended claims, "a", "an", and "the" in the singular form are intended to include the plural form, unless other circumstances are clearly indicated in the context.

It should be further understood that the term "and/or" used in the description of the present application and the appended claims refers to any combination and all possible combinations of one or more of the associated listed items, and includes these combinations.

As used in this description and the appended claims, the term "if" may, depending on the context, be interpreted as "when..." or "once" or "in response to determining" or "in response to detecting". Similarly, the phrase "if it is determined" or "if [the described condition or event] is detected" may, depending on the context, be interpreted as meaning "once it is determined" or "in response to determining" or "once [the described condition or event] is detected" or "in response to detecting [the described condition or event]".

Although the content of the present invention has already been presented in detail by means of the preferred embodiments above, it should be recognized that the above description should not be regarded as a restriction of the present invention. Obviously, the embodiments described are merely some, not all, of the embodiments of the present invention. All other embodiments obtained by those skilled in the art on the basis of the embodiments in the present invention without any creative effort are included within the scope of protection of the present invention. Various amendments and substitutions for the present invention will be obvious to those skilled in the art after perusal of the content above. Thus, the scope of protection of the present invention shall be defined by the attached claims.

## Claims

1. A computer-implemented method for performing measurement and classification for Computed Tomography Fractional Flow Reserve, comprising:
dividing lesions into different lesion types, according to branch involvement and calcification and length of lesions displayed in a Coronary Computed Tomography Angiography, CCTA, image (S1); **characterized in that** the method further comprises:
determining focus lengths of lesion positions according to the different lesion types (S2);
determining measurement ranges for Computed Tomography Fractional Flow Reserve according to the focus lengths of the different lesion types (S3);
obtaining all Computed Tomography Fractional Flow Reserve, CT-FFR, values within the measurement ranges, subjecting all the CT-FFR values to statistical calculation, and performing lesion specific classification of calculation results according to cut-off values from hemodynamical classification, to obtain a lesion specific classification result (S4).

2. The method for performing measurement and classification for Computed Tomography Fractional Flow Reserve as claimed in claim 1, **characterized in that** the method of dividing lesion types (S1) comprises:
if the length of a lesion is less than or equal to 10 mm, with mild or no calcification, and no principal branch involvement, then it is determined as being a single focal lesion;
if the length of a lesion is greater than or equal to 20 mm, with principal branch involvement, and excessive proximal twisting, then it is determined as being a diffuse lesion;
if the length of an entire lesion is more than 10 mm but less than 20 mm, with moderate or severe calcification, and an irregular contour, then it is determined as being a serial lesion; if lesions are spaced apart by less than 10 mm, then they are determined as being a continuous serial lesion, and if lesions are spaced apart by 10 mm or more, then they are determined as being a discontinuous serial lesion.

3. The method for performing measurement and classification for Computed Tomography Fractional Flow Reserve as claimed in claim 2, **characterized in that** the method of determining focus lengths of lesion positions (S2) comprises:
for a single focal lesion, separately recording a lesion proximal centerline point LPₚᵣₒ and a lesion distal centerline point LP_{dis};
for a diffuse lesion, separately recording a lesion proximal centerline point LPₚᵣₒ and a lesion distal centerline point LP_{dis};
for a serial lesion, first determining a proximal centerline point LPₚᵣₒ and a lesion distal centerline point LP_{dis} of the entire lesion, then determining continuity of the entire lesion, to determine whether the serial lesion is a continuous serial lesion or a discontinuous serial lesion;
for a continuous serial lesion, separately recording a lesion proximal centerline point LPₚᵣₒ and a lesion distal centerline point LP_{dis};
for a discontinuous serial lesion, first recording a lesion proximal centerline point LPₚᵣₒ and a lesion distal centerline point LP_{dis} of the entire lesion, then separately recording a lesion proximal centerline point LPₚᵣₒ' and a lesion distal centerline point LP_{dis}' of each lesion.

4. The method for performing measurement and classification for Computed Tomography Fractional Flow Reserve as claimed in claim 3, **characterized in that** for single focal lesions, diffuse lesions and continuous serial lesions, the method of determining the measurement range for Computed Tomography Fractional Flow Reserve (S3) comprises:
calculating a centerline point MP_{beg} for the start of measurement and a centerline point MP_{end} for the end of measurement: ${MP}_{beg} = {LP}_{dis} + 2 cm$ wherein the centerline point MP_{beg} for the start of measurement is 2 cm longitudinally along the coronary artery from the lesion distal centerline point LP_{dis} in the lesion distal direction; ${MP}_{end} = {LP}_{dis} + 3 cm$ wherein the centerline point MP_{end} for the end of measurement is 3 cm longitudinally along the coronary artery from the lesion distal centerline point LP_{dis} in the lesion distal direction.

5. The method for performing measurement and classification for Computed Tomography Fractional Flow Reserve as claimed in claim 3, **characterized in that** for discontinuous serial lesions, the method of determining the measurement range for Computed Tomography Fractional Flow Reserve (S3) comprises:
calculating a centerline point MP_{beg} for the start of measurement and a centerline point MP_{end} for the end of measurement: ${MP}_{beg} = {CLP}_{normal from {LP}_{dis}}$ wherein the centerline point MP_{beg} for the start of measurement is the position of the centerline point where normal blood vessel diameter is restored from the previous lesion; ${MP}_{end} = {MP}_{beg} + \left(0.5 to 1\right) mm$ wherein the centerline point MP_{end} for the end of measurement is 0.5 mm - 1 mm longitudinally along the coronary artery from the centerline point MP_{beg} for the start of measurement in the lesion distal direction.

6. The method for performing measurement and classification for Computed Tomography Fractional Flow Reserve as claimed in claim 4 or 5, **characterized in that** if a distal end of a lesion is close to the farthest end of the coronary artery, then a centerline point at a position where lumen diameter is 1.5 mm is taken to be the lesion distal centerline point when determining the focus length of the lesion position, and a centerline point at a position where lumen diameter is 1.5 mm is taken to be the centerline point MP_{end} for the end of measurement when determining the measurement range for Computed Tomography Fractional Flow Reserve: ${MP}_{end} = 1.5 mm lumen diameter centerline point .$

7. The method for performing measurement and classification for Computed Tomography Fractional Flow Reserve as claimed in claim 6, **characterized in that** the method of performing lesion specific classification (S4) comprises:
when the maximum value of all the CT-FFR values within the measurement range is less than or equal to a cut-off value 0.7, the mean value of all the CT-FFR values within the measurement range is used as the CT-FFR value of the lesion, and the lesion is labeled as positive;
when the minimum value of all the CT-FFR values within the measurement range is greater than or equal to a cut-off value 0.85, the mean value of all the CT-FFR values within the measurement range is used as the CT-FFR value of the lesion, and the lesion is labeled as negative;
when there are CT-FFR values within the measurement range that are distributed between 0.7 and 0.85, a histogram is established on the basis of all the CT-FFR values, a category with the highest frequency in the histogram is determined, and the mean value of CT-FFR values in this category is calculated and compared with the cut-off values; if the mean value is less than or equal to 0.7, the lesion is determined as being a positive lesion, and labeled as positive; if the mean value is greater than or equal to 0.85, the lesion is determined as being a negative lesion, and labeled as negative; if the mean value is greater than 0.7 but less than 0.85, the lesion is determined as being in a gray zone.

8. A system for performing measurement and classification for Computed Tomography Fractional Flow Reserve, used to realize the method for performing measurement and classification for Computed Tomography Fractional Flow Reserve as claimed in any one of claims 1 - 7, wherein the system comprises:
a lesion type classification module (101), for dividing lesions into different types, according to branch involvement and calcification and length of lesions displayed in a Coronary Computed Tomography Angiography, CCTA, image; **characterized in that** the system further comprises:
a focus length determining module (102), for determining focus lengths of lesion positions according to the different lesion types;
a measurement range determining module (103), for determining measurement ranges for Computed Tomography Fractional Flow Reserve according to the focus lengths of the different lesion types;
a CT-FFR value calculating module (104), for calculating all Computed Tomography Fractional Flow Reserve, CT-FFR, values within the measurement ranges;
a statistical analysis module (105), for subjecting the Computed Tomography Fractional Flow Reserve values to statistical analysis according to the measurement ranges, to obtain a lesion specific classification result.

9. A device for performing measurement and classification for Computed Tomography Fractional Flow Reserve, comprising: a memory, a processor, and a program stored on the memory and able to run on the processor, the program being configured to realize the method for performing measurement and classification for Computed Tomography Fractional Flow Reserve as claimed in any one of claims 1 - 7.

10. A storage medium, wherein a program is stored on the storage medium, and the program, when executed by a processor, realizes the method for performing measurement and classification for Computed Tomography Fractional Flow Reserve as claimed in any one of claims 1 - 7.

## Patentansprüche

1. Computerimplementiertes Verfahren zum Durchführen einer Messung und Klassifizierung für eine Computertomografie-basierte fraktionelle Flussreserve, umfassend:
Aufteilen von Läsionen in verschiedene Läsionstypen gemäß einer Zweigbeteiligung und einer Verkalkung und Länge von Läsionen, die in einem koronaren Computertomographieangiographie-Bild, CCTA-Bild, (S1) angezeigt werden; **dadurch gekennzeichnet, dass** das Verfahren ferner umfasst:
Ermitteln von Fokusgrößen von Läsionspositionen gemäß den verschiedenen Läsionstypen (S2);
Ermitteln von Messbereichen für die Computertomografie-basierte fraktionelle Flussreserve gemäß den Fokusgrößen der verschiedenen Läsionstypen (S3);
Erhalten aller Werte der Computertomografie-basierten fraktionellen Flussreserve, CT-FFR-Werte, innerhalb der Messbereiche, Unterziehen aller CT-FFR-Werte einer statistischen Berechnung und Durchführen einer läsionsspezifischen Klassifizierung von Berechnungsergebnissen gemäß Cut-off-Werten aus einer hämodynamischen Klassifizierung, um ein läsionsspezifisches Klassifizierungsergebnis (S4) zu erhalten.

2. Verfahren zum Durchführen einer Messung und Klassifizierung für eine Computertomographie-basierte fraktionelle Flussreserve nach Anspruch 1, **dadurch gekennzeichnet, dass** das Verfahren zum Aufteilen von Läsionstypen (S1) umfasst:
wenn die Länge einer Läsion kleiner als oder gleich 10 mm ist, mit einer leichten oder keiner Verkalkung und ohne eine prinzipielle Zweigbeteiligung, wird sie als eine einzelne fokale Läsion ermittelt;
wenn die Länge einer Läsion größer als oder gleich 20 mm ist, mit einer prinzipiellen Zweigbeteiligung und einer übermäßigen proximalen Verdrehung, wird sie als eine diffuse Läsion ermittelt;
wenn die Länge einer gesamten Läsion mehr als 10 mm, jedoch weniger als 20 mm mit einer mäßigen oder starken Verkalkung und einer unregelmäßigen Kontur beträgt, wird sie als serielle Läsion ermittelt; wenn die Läsionen weniger als 10 mm voneinander beabstandet sind, werden sie als kontinuierliche serielle Läsion ermittelt, und wenn die Läsionen 10 mm oder mehr voneinander beabstandet sind, werden sie als diskontinuierliche serielle Läsion ermittelt.

3. Verfahren zum Durchführen einer Messung und Klassifizierung für eine Computertomographie-basierte fraktionelle Flussreserve nach Anspruch 2, **dadurch gekennzeichnet, dass** das Verfahren zum Ermitteln von Fokusgrößen von Läsionspositionen (S2) umfasst:
für eine einzelne fokale Läsion, getrenntes Aufzeichnen eines läsionsnahen Mittellinienpunkts LPₚᵣₒ und eines läsionsfernen Mittellinienpunkts LP_{dis};
für eine diffuse Läsion, getrenntes Aufzeichnen eines läsionsnahen Mittellinienpunkts LPₚᵣₒ und eines läsionsfernen Mittellinienpunkts LP_{dis};
für eine serielle Läsion, zuerst Ermitteln eines läsionsnahen Mittellinienpunkts LPₚᵣₒ und eines läsionsfernen Mittellinienpunkts LP_{dis} der gesamten Läsion, danach Ermitteln der Kontinuität der gesamten Läsion, um zu ermitteln, ob die serielle Läsion eine kontinuierliche serielle Läsion oder eine diskontinuierliche serielle Läsion ist;
für eine kontinuierliche serielle Läsion, getrenntes Aufzeichnen eines läsionsnahen Mittelinienpunkts LPₚᵣₒ und eines läsionsfernen Mittellinienpunkts LP_{dis};
für eine diskontinuierliche serielle Läsion, zuerst Aufzeichnen eines läsionsnahen Mittellinienpunkts LPₚᵣₒ und eines läsionsfernen Mittellinienpunkts LP_{dis} der gesamten Läsion, danach getrenntes Aufzeichnen eines läsionsnahen Mittellinienpunkts LPₚᵣₒ' und eines läsionsfernen Mittellinienpunkts LP_{dis}' von jeder Läsion.

4. Verfahren zum Durchführen einer Messung und Klassifizierung für eine Computertomographie-basierte fraktionelle Flussreserve nach Anspruch 3, **dadurch gekennzeichnet, dass** das Verfahren zum Ermitteln des Messbereichs für die Computertomographie-basierte fraktionelle Flussreserve (S3) für einzelne fokale Läsionen, diffuse Läsionen und kontinuierliche serielle Läsionen umfasst:
Berechnen eines Mittellinienpunkts MP_{beg} für den Beginn einer Messung und eines Mittellinienpunkts MP_{end} für das Ende der Messung: ${MP}_{beg} = {LP}_{dis} + 2 cm$
wobei der Mittellinienpunkt MP_{beg} für den Beginn der Messung 2 cm in Längsrichtung entlang der Koronararterie von dem läsionsfernen Mittellinienpunkt LP_{dis} in der läsionsfernen Richtung liegt; ${MP}_{end} = {LP}_{dis} + 3 cm$
wobei der Mittellinienpunkt MP_{beg} für das Ende der Messung 3 cm in Längsrichtung entlang der Koronararterie von dem läsionsfernen Mittellinienpunkt LP_{dis} in der läsionsfernen Richtung liegt.

5. Verfahren zum Durchführen einer Messung und Klassifizierung für eine Computertomographie-basierte fraktionelle Flussreserve nach Anspruch 3, **dadurch gekennzeichnet, dass** das Verfahren zum Ermitteln des Messbereichs für eine Computertomographie-basierte fraktionelle Flussreserve (S3) für diskontinuierliche serielle Läsionen umfasst:
Berechnen eines Mittellinienpunkts MP_{beg} für den Beginn einer Messung und eines Mittellinienpunkts MP_{end} für das Ende der Messung: ${MP}_{beg} = {CLP}_{normal von {LP}_{dis}}$
wobei der Mittellinienpunkt MP_{beg} für den Beginn der Messung die Position des Mittellinienpunkts ist, an dem der normale Blutgefäßdurchmesser aus der vorherigen Läsion wiederhergestellt wurde; ${MP}_{end} = {MP}_{beg} + \left(0,5 bis 1\right) mm$
wobei der Mittellinienpunkt MP_{end} für das Ende der Messung 0,5 mm bis 1 mm in Längsrichtung entlang der Koronararterie von dem Mittellinienpunkt MP_{beg} für den Beginn der Messung in der läsionsfernen Richtung liegt.

6. Verfahren zum Durchführen einer Messung und Klassifizierung für eine Computertomographie-basierte fraktionelle Flussreserve nach Anspruch 4 oder 5, **dadurch gekennzeichnet, dass**, wenn sich ein fernes Ende einer Läsion in der Nähe des entferntesten Endes der Koronararterie befindet, ein Mittellinienpunkt an einer Position, an welcher der Lumendurchmesser 1,5 mm beträgt, als der läsionsferne Mittellinienpunkt angenommen wird, wenn die Fokusgröße der Läsionsposition ermittelt wird, und ein Mittellinienpunkt an einer Position, an welcher der Lumendurchmesser 1,5 mm beträgt, als der Mittellinienpunkt MP_{end} für das Ende der Messung angenommen wird, wenn der Messbereich für die Computertomographie-basierte fraktionelle Flussreserve ermittelt wird:
MP_{end} = Mittellinienpunkt mit einem 1,5-mm-Lumendurchmesser.

7. Verfahren zum Durchführen einer Messung und Klassifizierung für eine Computertomographie-basierte fraktionelle Flussreserve nach Anspruch 6, **dadurch gekennzeichnet, dass** das Verfahren zum Durchführen einer läsionsspezifischen Klassifizierung (S4) umfasst:
wenn der Maximalwert aller CT-FFR-Werte innerhalb des Messbereichs kleiner als oder gleich einem Cut-Off-Wert von 0,7 ist, Verwenden des Mittelwerts aller CT-FFR-Werte innerhalb des Messbereichs als den CT-FFR-Wert der Läsion und Markieren der Läsion als positiv;
wenn der Minimalwert aller CT-FFR-Werte innerhalb des Messbereichs größer als oder gleich einem Cut-Off-Wert von 0,85 ist, Verwenden des Mittelwerts aller CT-FFR-Werte innerhalb des Messbereichs als den CT-FFR-Wert der Läsion und Markieren der Läsion als negativ;
wenn CT-FFR-Werte innerhalb des Messbereichs vorhanden sind, die zwischen 0,7 und 0,85 liegen, Erstellen eines Histogramms auf der Grundlage aller CT-FFR-Werte, Ermitteln einer Kategorie mit der größten Häufigkeit in dem Histogramm und Berechnen des Mittelwerts der CT-FFR-Werte in dieser Kategorie und Vergleichen mit den Cut-Off-Werten; wenn der Mittelwert kleiner als oder gleich 0,7 ist, Ermitteln der Läsion als eine positive Läsion und als positiv Markieren; wenn der Mittelwert größer als oder gleich 0,85 ist, Ermitteln der Läsion als eine negative Läsion und als negativ Markieren; wenn der Mittelwert größer als 0,7, aber kleiner als 0,85 ist, Ermitteln der Läsion als in einer Grauzone befindlich.

8. System zum Durchführen einer Messung und Klassifizierung für eine Computertomographie-basierte fraktionelle Flussreserve, das verwendet wird, um das Verfahren zum Durchführen einer Messung und Klassifizierung für eine Computertomographie-basierte fraktionelle Flussreserve gemäß einem der Ansprüche 1 bis 7 durchzuführen, wobei das System umfasst:
ein Läsionstyp-Klassifizierungsmodul (101) zum Unterteilen von Läsionen in verschiedene Typen gemäß einer Zweigbeteiligung und einer Verkalkung und Länge von Läsionen, die in einem koronaren ComputertomographieAngiographie-Bild, CCTA-Bild, angezeigt werden;
**dadurch gekennzeichnet, dass** das System ferner umfasst:
ein Fokusgrößenermittlungsmodul (102) zum Ermitteln von Fokusgrößen von Läsionspositionen gemäß den verschiedenen Läsionstypen;
ein Messbereichsermittlungsmodul (103) zum Ermitteln von Messbereichen für die Computertomographie-basierte fraktionelle Flussreserve gemäß den Fokusgrößen der verschiedenen Läsionstypen;
ein CT-FFR-Werteberechnungsmodul (104) zum Berechnen aller Werte der Computertomographie-basierten fraktionellen Flussreserve, CT-FFR-Werte, innerhalb der Messbereiche;
ein statistisches Analysemodul (105), um die Werte der Computertomographie-basierten fraktionellen Flussreserve einer statistischen Analyse gemäß den Messbereichen zu unterziehen, um ein läsionsspezifisches Klassifizierungsergebnis zu erhalten.

9. Vorrichtung zum Durchführen einer Messung und Klassifizierung für eine Computertomographie-basierte fraktionelle Flussreserve, umfassend: einen Speicher, einen Prozessor und ein Programm, das in dem Speicher gespeichert ist und in der Lage ist, in dem Prozessor ausgeführt zu werden, wobei das Programm konfiguriert ist, um das Verfahren zum Durchführen einer Messung und Klassifizierung für eine Computertomographie-basierte fraktionelle Flussreserve nach einem der Ansprüche 1 bis 7 durchzuführen.

10. Speichermedium, wobei in dem Speichermedium ein Programm gespeichert ist,
und wobei das Programm, wenn es von einem Prozessor ausgeführt wird, das Verfahren zum Durchführen einer Messung und Klassifizierung für eine Computertomographie-basierte fraktionelle Flussreserve nach einem der Ansprüche 1 bis 7 durchführt.

## Revendications

1. Procédé exécuté par ordinateur permettant de mettre en œuvre une mesure et une classification pour une réserve de flux fractionnaire en tomodensitométrie, comprenant :
la division de lésions en différents types de lésions, selon l'implication des branches, et la calcification et la longueur des lésions affichées dans une image d'angiographie coronaire par tomodensitométrie, CCTA, (S1) ; **caractérisé en ce que** le procédé comprend en outre :
la détermination de longueurs focales de positions de lésions selon les différents types de lésions (S2) ;
la détermination de plages de mesure pour la réserve de flux fractionnaire en tomodensitométrie selon les longueurs focales des différents types de lésions (S3) ;
l'obtention de toutes les valeurs de réserve de flux fractionnaire en tomodensitométrie, CT-FFR, dans les plages de mesure, la soumission de toutes les valeurs CT-FFR à un calcul statistique, et la réalisation d'une classification spécifique des lésions de résultats de calcul selon des valeurs seuil provenant d'une classification hémodynamique, pour obtenir un résultat de classification spécifique des lésions (S4).

2. Procédé permettant de mettre en œuvre une mesure et une classification pour une réserve de flux fractionnaire en tomodensitométrie selon la revendication 1, **caractérisé en ce que** le procédé de division de types de lésions (S1) comprend :
si la longueur d'une lésion est inférieure ou égale à 10 mm, avec une calcification légère ou nulle, et sans implication d'une branche principale, alors elle est déterminée comme étant une lésion focale unique ;
si la longueur d'une lésion est supérieure ou égale à 20 mm, avec implication d'une branche principale et torsion proximale excessive, alors elle est déterminée comme étant une lésion diffuse ;
si la longueur d'une lésion entière est supérieure à 10 mm mais inférieure à 20 mm, avec une calcification modérée ou sévère, et un contour irrégulier, alors elle est déterminée comme étant une lésion sérielle ; si les lésions sont espacées de moins de 10 mm, alors elles sont déterminées comme étant une lésion sérielle continue, et si les lésions sont espacées de 10 mm ou plus, alors elles sont déterminées comme étant une lésion sérielle discontinue.

3. Procédé permettant de mettre en œuvre une mesure et une classification pour une réserve de flux fractionnaire en tomodensitométrie selon la revendication 2, **caractérisé en ce que** le procédé de détermination de longueurs focales de positions de lésions (S2) comprend :
pour une lésion focale unique, l'enregistrement séparé d'un point de ligne centrale proximal de lésion LPₚᵣₒ et d'un point de ligne centrale distal de lésion LP_{dis} ;
pour une lésion diffuse, l'enregistrement séparé d'un point de ligne centrale proximal de lésion LPₚᵣₒ et d'un point de ligne centrale distal de lésion LP_{dis} ;
pour une lésion sérielle, d'abord la détermination d'un point de ligne centrale proximal LPₚᵣₒ et d'un point de ligne centrale distal de lésion LP_{dis} de la lésion entière, puis la détermination de la continuité de la lésion entière, afin de déterminer si la lésion sérielle est une lésion sérielle continue ou une lésion sérielle discontinue ;
pour une lésion sérielle continue, l'enregistrement séparé d'un point de ligne centrale proximal de lésion LPₚᵣₒ et d'un point de ligne centrale distal de lésion LP_{dis} ;
pour une lésion sérielle discontinue, d'abord l'enregistrement d'un point de ligne centrale proximal de lésion LPₚᵣₒ et d'un point de ligne centrale distal de lésion LP_{dis} de la lésion entière, puis l'enregistrement séparé d'un point de ligne centrale proximal de lésion LPₚᵣₒ' et d'un point de ligne centrale distal de lésion LP_{dis}' de chaque lésion.

4. Procédé permettant de mettre en œuvre une mesure et une classification pour une réserve de flux fractionnaire en tomodensitométrie selon la revendication 3, **caractérisé en ce que** pour des lésions focales simples, des lésions diffuses et des lésions sérielles continues, le procédé de détermination de la plage de mesure pour la réserve de flux fractionnaire en tomodensitométrie (S3) comprend :
le calcul d'un point de ligne centrale MP_{beg} pour le début de la mesure et d'un point de ligne centrale MP_{end} pour la fin de la mesure : ${MP}_{beg} = {LP}_{dis} + 2 cm$
dans laquelle le point de ligne centrale MP_{beg} pour le début de la mesure est à 2 cm longitudinalement le long de l'artère coronaire à partir du point de ligne centrale distal de lésion LP_{dis} dans la direction distale de la lésion ; ${MP}_{end} = {LP}_{dis} + 3 cm$
dans laquelle le point de ligne centrale MP_{end} pour la fin de la mesure est à 3 cm longitudinalement le long de l'artère coronaire à partir du point de ligne centrale distal de lésion LP_{dis} dans la direction distale de la lésion.

5. Procédé permettant de mettre en œuvre une mesure et une classification pour une réserve de flux fractionnaire en tomodensitométrie selon la revendication 3, **caractérisé en ce que** pour des lésions sérielles discontinues, le procédé de détermination de la plage de mesure pour la réserve de flux fractionnaire en tomodensitométrie (S3) comprend :
le calcul d'un point de ligne centrale MP_{beg} pour le début de la mesure et d'un point de ligne centrale MP_{end} pour la fin de la mesure : ${MP}_{beg} = {CLP}_{normal from {LP}_{dis}}$
dans laquelle le point de ligne centrale MP_{beg} pour le début de la mesure est la position du point de ligne centrale où le diamètre normal de vaisseau sanguin est restauré à partir de la lésion précédente ; ${MP}_{end} = {MP}_{beg} + \left(0,5 à 1\right) mm$
dans laquelle le point de ligne centrale MP_{end} pour la fin de la mesure est à 0,5 mm à 1 mm longitudinalement le long de l'artère coronaire à partir du point de ligne centrale MP_{beg} pour le début de la mesure dans la direction distale de lésion.

6. Procédé permettant de mettre en œuvre une mesure et une classification pour une réserve de flux fractionnaire en tomodensitométrie selon la revendication 4 ou 5, **caractérisé en ce que** si une extrémité distale d'une lésion est proche de l'extrémité la plus éloignée de l'artère coronaire, alors un point de ligne centrale à une position où le diamètre de la lumière est de 1,5 mm est considéré comme le point de ligne centrale distal de la lésion lors de la détermination de la longueur focale de la position de la lésion, et un point de ligne centrale à une position où le diamètre de la lumière est de 1,5 mm est considéré comme le point de ligne centrale MP_{end} pour la fin de la mesure lors de la détermination de la plage de mesure pour la réserve de flux fractionnaire en tomodensitométrie :
MP_{end} = point de ligne centrale de diamètre de lumière de 1,5 mm.

7. Procédé permettant de mettre en œuvre une mesure et une classification pour une réserve de flux fractionnaire en tomodensitométrie selon la revendication 6, **caractérisé en ce que** le procédé de réalisation d'une classification spécifique de lésion (S4) comprend :
lorsque la valeur maximale de toutes les valeurs CT-FFR dans la plage de mesure est inférieure ou égale à une valeur seuil de 0,7, la valeur moyenne de toutes les valeurs CT-FFR dans la plage de mesure est utilisée comme valeur CT-FFR de la lésion, et la lésion est étiquetée comme positive ;
lorsque la valeur minimale de toutes les valeurs CT-FFR dans la plage de mesure est supérieure ou égale à une valeur seuil de 0,85, la valeur moyenne de toutes les valeurs CT-FFR dans la plage de mesure est utilisée comme valeur CT-FFR de la lésion, et la lésion est étiquetée comme négative ;
lorsqu'il y a des valeurs CT-FFR dans la plage de mesure qui sont réparties entre 0,7 et 0,85, un histogramme est établi sur la base de toutes les valeurs CT-FFR, une catégorie ayant la fréquence la plus élevée dans l'histogramme est déterminée, et la valeur moyenne des valeurs CT-FFR dans cette catégorie est calculée et comparée aux valeurs de seuil ; si la valeur moyenne est inférieure ou égale à 0,7, la lésion est déterminée comme étant une lésion positive, et marquée comme positive ; si la valeur moyenne est supérieure ou égale à 0,85, la lésion est déterminée comme étant une lésion négative, et marquée comme négative ; si la valeur moyenne est supérieure à 0,7 mais inférieure à 0,85, la lésion est déterminée comme étant dans une zone grise.

8. Système pour mettre en œuvre une mesure et une classification pour une réserve de flux fractionnaire en tomodensitométrie, utilisé pour réaliser le procédé permettant de mettre en œuvre une mesure et une classification pour une réserve de flux fractionnaire en tomodensitométrie selon l'une quelconque des revendications 1 à 7, dans lequel le système comprend :
un module de classification de types de lésions (101), pour diviser des lésions en différents types, selon l'implication de branche et la calcification et la longueur de lésions affichées dans une image d'angiographie coronaire par tomodensitométrie, CCTA ;
**caractérisé en ce que** le système comprend en outre :
un module de détermination de longueur focale (102), pour déterminer des longueurs focales de positions de lésions selon les différents types de lésions ;
un module de détermination de plages de mesure (103), pour déterminer des plages de mesure pour la réserve de flux fractionnaire en tomodensitométrie selon les longueurs focales de différents types de lésions ;
un module de calcul de valeurs CT-FFR (104), pour calculer toutes les valeurs de réserve de flux fractionnaire en tomodensitométrie, CT-FFR, à l'intérieur des plages de mesure ;
un module d'analyse statistique (105), pour soumettre les valeurs de réserve de flux fractionnaire de tomodensitométrie à une analyse statistique en fonction des plages de mesure, afin d'obtenir un résultat de classification spécifique des lésions.

9. Dispositif pour mettre en œuvre une mesure et une classification pour une réserve de flux fractionnaire en tomodensitométrie, comprenant : une mémoire, un processeur, et un programme stocké dans la mémoire et pouvant s'exécuter sur le processeur, le programme étant configuré pour réaliser le procédé permettant de mettre en œuvre une mesure et une classification pour une réserve de flux fractionnaire en tomodensitométrie selon l'une quelconque des revendications 1 à 7.

10. Support de stockage, un programme étant stocké sur le support de stockage,
et le programme, lorsqu'il est exécuté par un processeur, réalise le procédé permettant de mettre en œuvre une mesure et une classification pour une réserve de flux fractionnaire en tomodensitométrie selon l'une quelconque des revendications 1 à 7.
